# EUROPÄISCHE PATENTSCHRIFT

(11) **EP 0 852 150 B1**
(45) Veröffentlichungstag und Bekanntmachung des Hinweises auf die Patenterteilung: **19.03.2003**
(21) Anmeldenummer: 97119489.9
(22) Anmeldetag: 07.11.1997
(51) Int. Cl.: A61M 1/00, A61M 1/36, B01D 19/00

(54) **Blut-Gas-Trennvorrichtung**
Blood/gas separator
Dispositif de séparation pour sang/gaz

(30) Priorität: 05.12.1996 DE 19650407
(43) Veröffentlichungstag der Anmeldung: 08.07.1998
(73) Patentinhaber: Convergenza AG, 9490 Vaduz (LI)
(72) Erfinder: Brockhoff, Alexander, 9494 Schaan (LI); Plechinger, Hans, Cranbrook, B.C. VIC 6J5, CANADA (CA)
(74) Vertreter: Vetter, Ewald Otto, Dipl.-Ing.

(56) Entgegenhaltungen:
- EP-A- 0 318 993
- EP-A- 0 778 031
- DE-A- 2 611 383
- DE-A- 4 326 886
- DE-A- 4 329 385
- US-A- 3 785 380
- US-A- 4 710 299

## Beschreibung

Die Erfindung betrifft eine Vorrichtung zum Trennen von Gas aus Blut gemäß dem Oberbegriff von Anspruch 1.

Eine Vorrichtung dieser Art zum Trennen von Gas aus Blut ist aus der DE 43 29 385 A1 bekannt. Sie hat eine zylindrische Zentrifugierkammer. Im Zentrum ihres oberen Bereiches ist ein Blut-Einlaufverteiler mit mehreren Leitschaufeln angeordnet, welcher einen Blutstrom auf die Leitschaufeln aufteilt. Die einzelnen Blutströme werden von dem Einlaufverteiler ungefähr tangential an die zylindrische Wand der Zentrifugierkammer getrieben. Über diesem Bluteinlaufbereich befindet sich in der Zentrifugierkammer ein Luftpolster, aus welchem aus dem Blut aufsteigende Luftblasen durch eine Entlüftungsbohrung abgeleitet werden können. Im Boden der Zentrifugierkammer befindet sich eine Blutauslaßöffhung.

Die US-A-4 710 299 zeigt einen allgemein verwendbaren Zyklonseparator zum Trennen dichter Komponenten einer Fluidmischung von weniger dichten Komponenten davon. Ihre Zentrifugierkammer hat aufeinanderfolgende konische und zylindrische Abschnitte. Ein Einlaßkanal, welcher im Wesentlichen tangential und gleichzeitig schräg von oben nach unten in die Zentrifugierkammer mündet, hat einen in Strömungsrichtung stark enger werdenden Querschnitt zur Erzeugung einer Strömungsbeschleunigung. Die dichteren Komponenten werden durch eine Auslaßöffnung im Kammerboden, und die weniger dichten Komponenten werden durch eine Auslaßöffnung in der Kammerdecke je axial aus der Zentrifugierkammer herausgeführt.

Wenn von einem Patienten Blut abgesaugt wird, insbesondere von einer Wundstelle des Patienten, welche durch eine Operation oder einen Unfall entstehen kann, und dieses Blut anschließend für den gleichen Patienten oder einen anderen Patienten wieder verwendet werden soll, dann muß das Blut möglichst sofort und möglichst nahe bei der Absaugstelle des Patienten wieder von der Luft getrennt werden, welche gegebenenfalls an der Absaugstelle des Patienten in das Blut eingesaugt wurde. Einem Patienten darf kein Blut zugeführt werden, welches Luft enthält. Außerdem wird das Blut durch Lufteinschlüsse geschädigt, je länger eingesaugte Luft im Blut verbleibt und je stärker die Luft auf dem Blutabsaugweg mit diesem Blut vermischt wird.

Die Qualität von Blut kann unter anderem beeinträchtigt werden: durch die Menge und die Zeitdauer von Luft oder anderen Gasen im Blut; durch Saug- oder Druckkräfte auf das Blut; durch Reibungskräfte des Blutes in Strömungswegen; Umlenkungen der Blutströmung und Turbulenzen im Blut.

Beim Entgasen von Blut müssen verschiedene Anwendungsfälle unterschieden werden, da sie verschiedene Aufgaben und verschiedene Bedingungen haben:

**1. Anwendungsfall:** Das Entgasen von Blut, während es in verhältnismäßig kleinen und stark sich ändernden Mengen von einem Patienten abgesaugt wird, insbesondere durch eine Saugkanüle von einer Wundstelle des Patienten, welche durch einen Unfall oder während einer Operation entstehen kann. Der Unterdruck zum Absaugen des Blutes wird durch eine Saugpumpe, meistens eine Rollerpumpe, erzeugt. Die Saugpumpe saugt nicht nur Blut, sondern an der Blutabsaugstelle des Patienten häufig auch Luft ab. Der abgesaugte Luftanteil ist verhältnismäßig hoch zu dem abgesaugten Blutanteil, beispielsweise 5 Teile Luft zu einem Teil Blut (Volumenteile). Die Blutabsaugrate ist verhältnismäßig niedrig, beispielsweise 100 bis 600 Milliliter pro Minute, und schwankt stark. Die Absaug-Strömungsgeschwindigkeit des Blutes ist ebenfalls verhältnismäßig niedrig und schwankt stark.

Die im Blut enthaltenen Luftbläschen sind häufig verhältnismäßig groß. Ihre Größe reicht vom Mikrometerbereich bis zum Millimeterbereich. Die vorliegende Erfindung betrifft diesen Anwendungsbereich, nämlich das Entgasen von Blut, während es von einem Patienten abgesaugt wird.

**2. Anwendungsfall:** Das Zuführen von Blut zu einem Patienten, beispielsweise bei der Blutwäsche (Dialyse) oder bei hohem Blutverlust bei einem Unfall. Blut, welches einem Patienten zugeführt wird, kann nicht gesaugt werden, sondern kann nur durch Druck einer Druckpumpe dem Patienten zugeführt werden. Die Blutrate, beispielsweise 3 Liter pro Minute, und die Fördergeschwindigkeit des Blutes sind verhältnismäßig hoch und im wesentlichen konstant. Der Luftanteil im Blut ist jedoch gering, beispielsweise nur 50 x 10⁻⁶ Volumenteile Luft zu einem Volumenteil Blut. Die Luft hat die Form von nur sehr kleinen Bläschen im Mikrometerbereich.

**3. Anwendungsfall:** Die Behandlung von Blut außerhalb und unabhängig von einem Patienten. Hier hat man ähnlich wie beim 2. Anwendungsfall im wesentlichen konstante Blutströmungsmengen und konstante Blutströmungsgeschwindigkeiten.

Zum ersten Anwendungsfall ist aus der US-A-3 785 380 eine Blutabsaugvorrichtung bekannt, welche aus einem zylindrischen Gehäuse, in welchem sich mikroporöses Filtermaterial zum Ausfiltern von Luftbläschen und anderen Verunreinigungen aus einem von einem Patienten abgesaugten Blutstrom befinden, einer Blutabsaugkanüle an einem vorderen Ende des Gehäuses und aus einer Blutabsaugleitung am hinteren Ende des Gehäuses besteht.

Zum genannten zweiten Anwendungsfall ist folgende Literatur bekannt: GB-A-2 063 108 zeigt eine Blutentgasungsvorrichtung zum Entfernen von Gasblasen, welche so klein sein können, daß sie im Mikrobereich liegen, beispielsweise einen Durchmesser von nur 40 Mikron haben. Die Blutabsaugvorrichtung besteht aus einer vertikal angeordneten zylindrischen Zyklonkammer, einem tangentialen Einlaß am oberen Kammerende, einem entgegengesetzt zur Zyklon-Rotationsbewegung tangential angeordneten Blutauslaß am unteren Kammerende, ein Entlüftungsrohr, welches in der Zyklon-Rotationsachse von oben nach unten bis unterhalb des Bluteinlasses in die Zyklonkammer hineinragt, einem zweiten Entlüftungsmittel in Form einer radialen Bohrung in einer oberen Verlängerung der Zyklonkammer oberhalb des Bluteinlasses, und einem zweiten Rohr, welches sich längs der Zyklon-Rotationsachse durch die gesamte Zyklonkammer und durch einen Teil des erstgenannten Rohres erstreckt und dazu dienen soll, daß an seiner Außenfläche Luftblasen sich sammeln und nach oben steigen können. Die DE-A-43 29 385 beschreibt, daß sie einen gegenüber der genannten GB-A-2 063 108 weiterentwickelten Luftabscheider betrifft, bei welchem Bluteinlaß und Blutauslaß an den voneinander angewandten Enden einer zylindrischen Wirbelkammer axial zueinander angeordnet sind. Der Bluteinlaß ist durch einen Leitschaufel-Körper gebildet und vor dem Blutauslaß befindet sich eine Filterkerze. Aufsteigende Blutbläschen gelangen in einen oberhalb des Leitschaufel-Körpers gelegenen Abschnitt der Wirbelkammer, wo ein Luftpolster gebildet ist, welches durch eine Bohrung entlüftet wird. Die DE-C-36 41 644 zeigt eine Blut-Durchflußkammer mit einem Bluteinlaß auf halber Kammerhöhe und einem in die Durchflußkammer eingetauchten Blutauslaßkanal. Im Blut enthaltene Luftblasen können nur durch die Auftriebskraft nach Archimedes nach oben steigen. Ferner zeigen die DE-C-36 24 363 und die US-A-5 451 321 Vorrichtungen mit mikroporösem Filtermaterial zum Filtern von Gasblasen oder anderen Blutverunreinigungen aus einem Blutstrom.

Durch die Erfindung soll die Aufgabe gelöst werden, eine Vorrichtung so zu gestalten, daß auch bei kleinen und dabei schwankenden Blutstrommengen ein schwacher Sog oder Unterdruck ausreicht, um das Blut so durch eine nicht-rotierende Zentrifugierkammer oder Zyklonkammer zu saugen, daß eine gute und schnelle Trennung von Blut und mit ihm vermischtem Gas, insbesondere Luft, bewirkt wird.

Diese Aufgabe wird gemäß der Erfindung durch die Merkmale von Anspruch 1 gelöst.

Die Erfindung hat insbesondere folgende Vorteile: Die Erfindung nützt zur Ausscheidung von Glasbläschen jeder Größe die Fliehkräfte einer Zyklonströmung, die Gasabsaug-Sogwirkung von oben auf das rotierende Blut, und die Auftriebskräfte nach Archimedes gleichzeitig und einander unterstützend aus, so daß bereits bei einem schwachen Sog einer Saugpumpe eine schonende und gleichzeitig wirksame Trennung der Gasphase aus der Blutphase stattfindet. Die nicht-rotierende Zentrifugierkammer oder Zyklonkammer hat die Form eines vom Bluteinlaß bis zum Blutauslaß nach unten hin enger werdenden Trichters, so daß die zentrifugale Bewegungsenergie der Zyklonströmung vom Bluteinlaß bis zum Blutauslaß besser aufrechterhalten wird als bei einer nicht-trichterförmigen, zylindrischen Kammer. Dadurch wird gemäß der Erfindung auch bei kleinen Blutströmungsmengen und niedrigen Blutströmungsgeschwindigkeiten noch eine Blutrotation mit ausreichender Fliehkraft zum Ausscheiden von Luft aus dem Blut erzeugt, selbst bei einem schwachen Sog der Saugpumpe. Der Wirkungsgrad wird zusätzlich verbessert, indem der Bluteinlaßkanal nicht nur ungefähr tangential, sondern in Blutströmungsrichtung auch schräg von oben nach unten in die Zyklonkammer gerichtet ist. Dadurch werden auch tropfenförmig kleine Blutmengen in der Kammer durch den Sog der Blut-Saugpumpe rotiert und von Luftbläschen befreit. Eine bessere Trennung von Blut und Gas aus dem Blut-Gas-Saugstrom wird gemäß einem besonderen Gedanken der Erfindung durch einen Gasraum über dem rotierenden Blutstrom erreicht, in welchem Gasblasen und Blut-Gas-Schaum genügend Zeit und Raum haben, um in einen Luftanteil und einen Blutanteil zu zerfallen, bevor das Gas mit Abstand oberhalb des rotierenden Blutstromes von einer Gas-Absaugvorrichtung abgesaugt wird. Die Erfindung wird vorzugsweise für den genannten 1. Anwendungsfall im Blutabsaugstrom eines Patienten angewendet. Die Erfindung kann jedoch auch gemäß dem 3. Anwendungsfall zur wirksamen Trennung von Gas aus Blut getrennt und unabhängig von einem Patienten angewendet werden, indem aus einem Blutbehälter Blut durch die Zentrifugierkammer oder Zyklonkammer gesaugt wird. Die Vorrichtung kann nahe bei einem Patienten angeordnet werden; sie ist gewichtsmäßig sehr leicht; sie kann als tragbares Ein-Hand-Gerät ausgebildet werden; sie kann unter Verwendung handelsüblicher Kanülen preiswert hergestellt werden; ihre Handhabung ist einfach und für die Bedienungsperson nicht ermüdend.

Weitere Merkmale und Vorteile der Erfindung ergeben sich dem Fachmann aus den Unteransprüchen.

Die Erfindung wird im folgenden mit Bezug auf die Zeichnungen anhand einer bevorzugten Ausführungsform als Beispiel beschrieben. In den Zeichnungen zeigen
- Fig. 1: eine Seitenansicht, teilweise längs der Ebene I-I von Fig. 3 geschnitten dargestellt, einer Blutabsaugvorrichtung gemäß der Erfindung im Maßstab 1:1,
- Fig. 2: eine schematische Längsschnitt-Ansicht längs der Ebene II-II von Fig. 1,
- Fig. 3: eine Draufsicht von oben auf die Blutabsaugvorrichtung von Fig. 1,
- Fig. 4: eine Seitenansicht, teilweise geschnitten, einer weiteren Ausführungsform nach der Erfindung,
- Fig. 5: eine Seitenansicht, teilweise geschnitten, einer nochmals weiteren Ausführungsform der Erfindung.

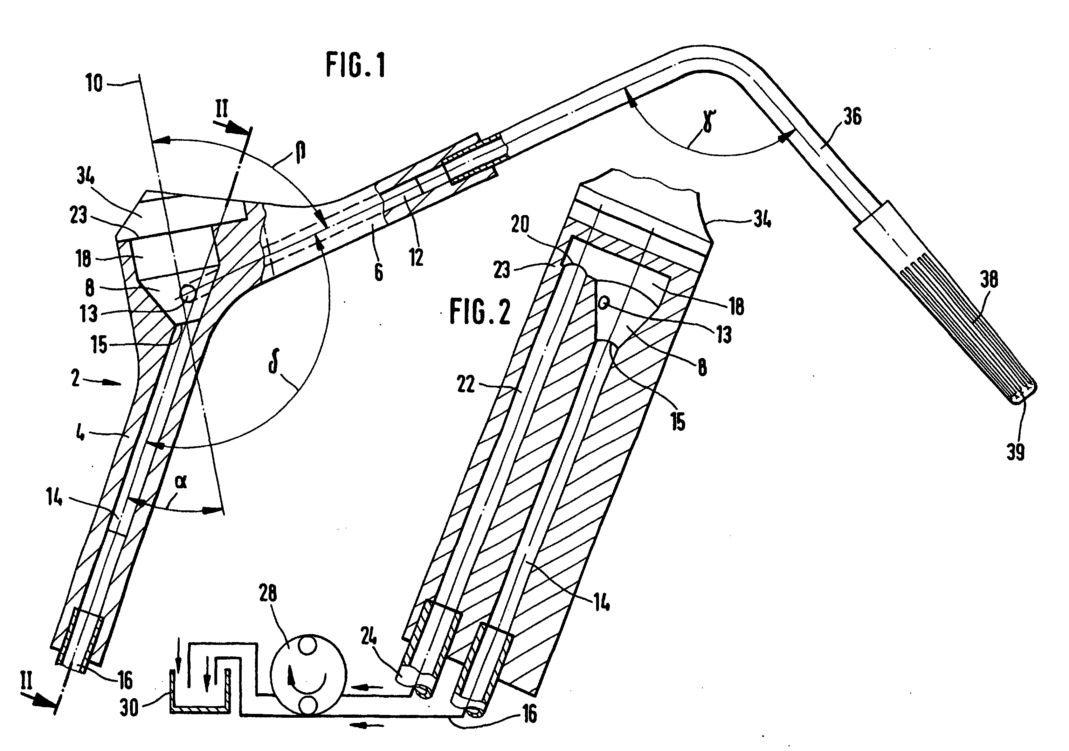

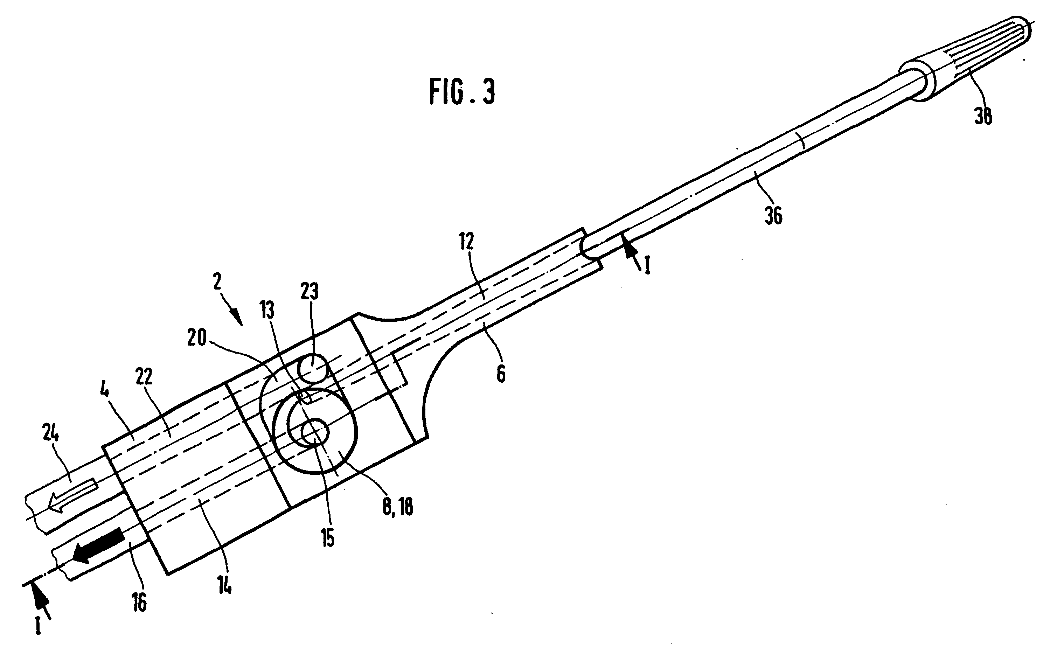

Die in den Fig. 1 bis 3 dargestellte Ausführungsform einer Blutabsaugvorrichtung nach der Erfindung zum Absaugen von Blut von einem Patienten, insbesondere von einer Wundstelle des Patienten, ist ein Ein-Hand-Gerät und enthält einen einteiligen oder mehrteiligen, pistolenförmigen Gerätekörper 2 aus Metall oder Kunststoff, der im wesentlichen aus einem Griff 4 und einem Lauf oder Schaft 6 besteht. Im Kreuzungsbereich zwischen Griff 4 und Schaft 6 ist im Gerätekörper 2 eine nicht-rotierende Zentrifugierkammer, im folgenden Zyklonkammer 8 genannt, zum Erzeugen eines mit gleichbleibender Drehrichtung zyklonartig rotierenden Blutstromes um eine aufrechte, vertikale oder nahezu vertikale Rotationsachse 10 gebildet. Die Zyklonkammer 8 hat von oben nach unten eine trichterartig enger werdende Form, damit ein durch sie hindurch gesaugter Blutstrom durch die ganze Zyklonkammer hindurch seine Bewegungsenergie im wesentlichen aufrechterhält, ohne daß eine starke Saugkraft in der Zyklonkammer erforderlich ist. Ein Bluteinlaß-Saugkanal 12 im Schaft 6 mündet bei einer Bluteinlaßöffnung 13 ungefähr tangential, und unter einem Winkel β von 90° oder vorzugsweise von weniger als 90° zur vertikalen Rotationsachse 10 schräg von oben nach unten, in den trichterförmigen oberen Abschnitt der trichterförmigen Zyklonkammer 8. Ein Blutauslaß-Saugkanal 14 erstreckt sich von einer Blutauslaßöffnung 15 am engen unteren Ende der Zyklonkammer 8 unter einem, nach unten offenen, Winkel α zwischen 0° und 90°, vorzugsweise ungefähr 30°, zur Rotationsachse 10 durch den Griff 4 zu einem an sein unteres Ende angeschlossenen Blutabsaugschlauch 16. Damit erstreckt sich der Blutauslaß-Saugkanal 14 entweder axial zur Rotationsachse 10 oder schräg von ihr weg von oben vorne nach unten hinten. Auf dem Strömungsweg zwischen dem Bluteinlaß-Saugkanal 12 und dem Blutauslaß-Saugkanal 14 rotiert der Blutstrom, ohne Umkehrströmungen, nur in einer einzigen Wirbel-Rotationsrichtung.

Die Zyklonkammer 8 ist durch einen über ihr angeordneten geschlossenen Gasraum 18 von ungefähr 5mm bis 15mm Höhe nach oben verlängert, welcher einen über die Zyklonkammer 8 seitlich hinausragenden Gasraumabschnitt 20 hat. Der Gasraum 18 dient als Speicherkammer zur temporären Aufnahme von Gasbläschen und Blutschaum, so daß sie Zeit und Raum zum Zerfallen in Luft und Blut haben. Dadurch wird der Blutanteil reduziert, der mit dem Gas, getrennt vom rotierenden Blutstrom abgesaugt wird. Der Gasraum 18 dient auch zur temporären Aufnahme von gegebenenfalls zeitweise aus der Zyklonkammer 8 aufsteigendem Blut. Der Querschnitt des Gasraumes 18 quer zur Rotationsachse 10 ist mindestens zweimal so groß wie der Querschnitt einer Gasauslaßöffnung 23.

Durch den Griff 4 erstreckt sich parallel zum Blutauslaß-Saugkanal 14 ein Gasauslaß-Saugkanal 22, der über die Gasauslaßöffnung 23 im Boden des seitlich überstehenden Gasraumabschnittes 20 mit dem Gasraum 18 verbunden ist. Die Gasauslaßöffnung 23 ist wesentlich höher angeordnet, z.B. 2mm - 10mm höher, als die Bluteinlaßöffnung 13. Am unteren Ende des Griffes 4 ist an den Gasauslaß-Saugkanal 22 ein Gasabsaugschlauch 24 angeschlossen. Der Gasabsaugschlauch 24 und der Blutabsaugschlauch 16 sind getrennt an eine Saugpumpe 28 angeschlossen, vorzugsweise an eine peristaltische Rollerpumpe, welche das abgesaugte Blut und die abgesaugte Luft auf getrennten Wegen in ein Blutreservoir 30 fördert. Dabei erzeugt die Saugpumpe 28 über den Blutauslaß-Saugkanal 14 und den Gasauslaß-Saugkanal 22 durch den Gasraum 18 und die Zyklonkammer 8 hindurch einen Saug-Unterdruck im Bluteinlaß-Saugkanal 12, so daß durch den Bluteinlaß-Saugkanal 12 Blut in die Zyklonkammer 8 gesaugt wird.

Der Gasraum 18 ist durch einen Deckel 34 geschlossen.

In das distale (vordere) Ende des Bluteinlaß-Saugkanals 6 ist eine Blutabsaug-Kanüle 36 lösbar gesteckt. Gemäß einer anderen Ausführungsform kann die Blutabsaug-Kanüle 36 auch ein einstückiges Teil mit dem Gerätekörper 2 bilden. Der vordere Endabschnitt 38 der Blutabsaugkanüle 36 ist um einen nach unten offenen Winkel γ von beispielsweise 105° nach unten abgewinkelt und mit Durchlaßöffnungen 39 versehen, um von einer Wundstelle eines Patienten Blut absaugen zu können, wenn eine Bedienungsperson den Gerätekörper 2 am Handgriff 4 in bequemer Handposition hält. Dementsprechend kann der Winkel y der Blutabsaugkanüle 36 im Bereich zwischen 90° und 180° liegen.

Der Griff 4 und in ihm der Blutauslaß-Saugkanal 14 und der zu ihm parallele Gasauslaß-Saugkanal 22 erstrecken sich unter einem nach unten offenen Winkel α zwischen 0° und 90°, vorzugsweise ungefähr 30°, relativ zur Zyklon-Rotationsachse 10 schräg nach unten hinten. Die Winkel α und γ sind so aufeinander abgestimmt, daß die Vorrichtung bei der Blutabsaugung bequem am Griff getragen werden kann und dabei die Gasauslaßöffnung 23 immer höher angeordnet bleibt als die Bluteinlaßöffnung 13. Deshalb ist die Gasauslaßöffnung 23 vorzugsweise auf der von der Bluteinlaßöffnung 13 abgewandten Kammerseite angeordnet.

Der nach unten offene Winkel δ zwischen dem Blutauslaß-Saugkanal 14 und dem Gasauslaß-Saugkanal 22 einerseits und dem Bluteinlaß-Saugkanal 12 andererseits liegt, abhängig von den Winkeln α und β zwischen ungefähr 90° und 180°, vorzugsweise gemäß der dargestellten bevorzugten Ausführungsform bei 135°.

Gemäß der bevorzugten Ausführungsform haben der Gasauslaß-Saugkanal 22 und sein Gasabsaugschlauch 24 einen gleichen oder anderen Innenquerschnitt wie oder als der Blutabsaugkanal 14 und sein Blutabsaugschlauch 16, so daß Blut und Luft getrennt voneinander von der gleichen Saugpumpe 28 abgesaugt werden können, auch dann, wenn das Blut und die Luft verschiedene Strömungsvolumen haben.

Die von der Nähe der Bluteinlaßöffnung 13 bis zur Nähe der Blutauslaßöffnung 15 von oben nach unten enger werdende Konusform oder Trichterform der Zyklonkammer 8 gewährleistet, daß die Rotationsenergie des Blutstromes vom Bluteinlaß-Saugkanal 12 bis zum Blutauslaß-Saugkanal 14 ohne wesentliche Verlust aufrechterhalten wird, selbst wenn am Blutauslaß 15 nur ein geringer, das Blut schonender, Sog von der Saugpumpe 28 erzeugt wird.

Wenn der stromabwärtige Endabschnitt des Bluteinlaß-Saugkanals 12 bei der Bluteinlaßöffnung 13 nicht nur im wesentlichen tangential, sondern auch schräg von oben nach unten unter einem Winkel β von weniger als 90° in die Zyklonkammer 18 gerichtet ist, und damit in Richtung des Soges im Blutauslaß-Saugkanal 14 geneigt ist, dann werden von diesem Sog auch tropfenartig kleine Blutmengen in der Zyklonkammer 18 so schnell rotiert, daß Blut und Luft trennende Fliehkräfte entstehen.

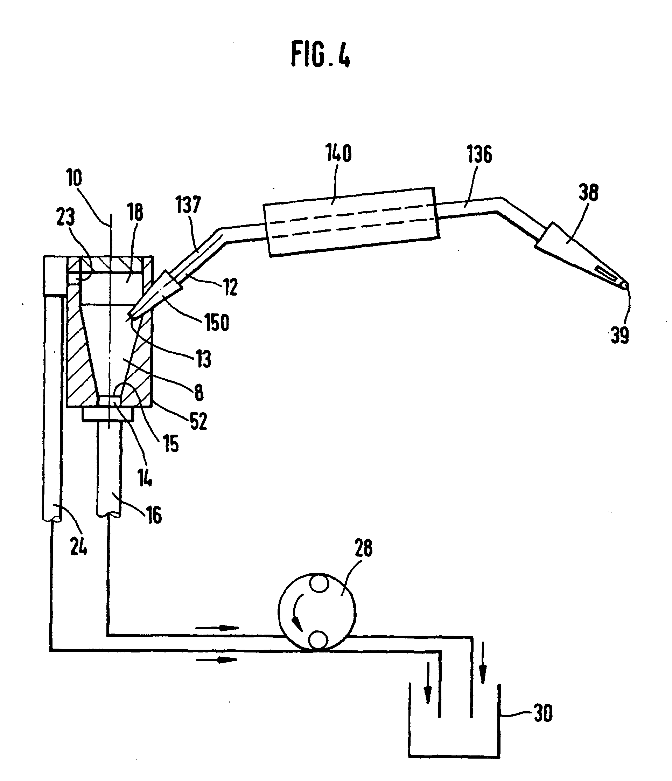

Bei der in Fig. 4 dargestellten Ausführungsform nach der Erfindung wird eine handelsübliche Blutabsaugkanüle 136 mit einem Handgriffteil 140 verwendet. Der stromabwärtige, hintere Endabschnitt 137 dieser Blutabsaugkanüle 136 bildet direkt oder durch ein Anschlußstück 150 den Bluteinlaß-Saugkanal 12 mit der Bluteinlaß-Öffnung 13 in der Zyklonkammer 8. Die Zyklonkammer 8 und der sich an ihr oberes Ende anschließende, nach oben erstreckende Gasraum 18 sind in einem Gehäuse 52 gebildet. Die weiteren in Fig. 4 gezeigten Details sind konstruktiv und zumindest funktionell identisch mit den Details der Fig. 1 bis 3 und sind mit gleichen Bezugszahlen versehen.

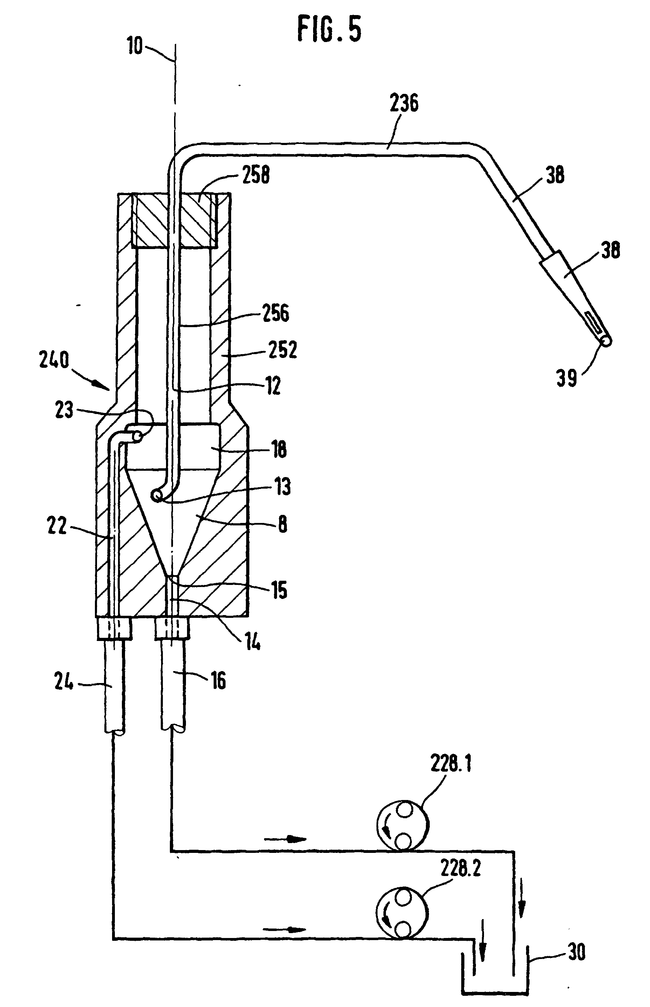

Bei der weiteren Ausführungsform der Erfindung gemäß Fig. 5 sind die Zyklonkammer 8 und der Gasraum 18 in einem Gehäuse 252 gebildet, welches gleichzeitig als Handgriffteil 240 ausgebildet ist, an welchem das gesamte Gerät mit einer Hand getragen werden kann. Die Blutabsaugkanüle 236 hat an ihrem stromabwärtigen, hinteren Ende ein vertikal von oben nach unten in das Gehäuse 252 hinein reichendes Rohrstück 256, welches den Bluteinlaß-Saugkanal 12 und, an seinem stromabwärtigen Ende, die Bluteinlaßöffnung 13 bildet. Der Gasraum 18 ist nach oben durch einen Verschluß 258 am oberen Ende des Gehäuses 252 verschlossen. Es ist eine Saugpumpe 228.1 für das Blut und eine Saugpumpe 228.2 für das Gas vorgesehen, jedoch können beide durch eine einzige Pumpe 28 entsprechend den Fig. 1 bis 4 ersetzt werden. Das Vakuum dieser Pumpen erzeugt durch die Gasauslaßöffnung 23, die Blutauslaßöffnung 15, die Zyklonkammer 18 und durch die Blutabsaugkanüle 236 hindurch einen Unterdruck oder Sog, durch welchen Blut von der Wundstelle eines Patienten in Öffnungen 39 am vorderen Katheterende eingesaugt wird. Die weiteren Details der Ausführungsform nach Fig. 5 sind gleich wie bei der Ausführungsform nach den Fig. 1 bis 3 und mit den gleichen Bezugszahlen versehen.

Bei den Fig. 4 und 5 fluchten die Blutauslaß-Saugkanäle 14 mit der vertikalen Rotationsachse 10 der als Zentrifugierkammer wirkenden Zyklonkammer 8.

## Patentansprüche

1. Vorrichtung zum Trennen von Gas aus Blut, enthaltend
1.1 eine nicht-rotierende Zentrifugierkammer (8) zum Erzeugen eines mit gleichbleibender Drehrichtung zyklonartig um eine durch die Zentrifugierkammer (8) definierte, aufrechte, vertikale oder nahezu vertikale Rotationsachse (10) rotierenden Blutstromes;
1.2 einen Bluteinlaß (12, 13) im oberen Bereich und eine Blutauslaßöffnung (15) im unteren Bereich der Zentrifugierkammer (8), um einen Blutstrom von dem Sog oder dem Unterdruck einer an die Blutauslassöffnung (15) anschließbaren Saugvorrichtung (28) von einer Blutquelle über den Bluteinlaß (12, 13) durch die Zentrifugierkammer (8), in welcher er um die Rotatiosachse rotiert, ohne Umkehrung der Blutstrom-Rotationsrichtung durch die Blutauslaßöffnung (15) zu saugen;
1.3 wobei die Strömungsrichtung des Bluteinlasses (12, 13) im wesentlichen tangential zur Rotationsachse (10) des Blutstromes in die Zentrifugierkammer gerichtet ist;
1.4 ein mit Höhenabstand höher als der Bluteinlass (12, 13) angeordneter Gasausläß (23);
**dadurch gekennzeichnet**,
1.5 dass der Bluteinlaß ein Bluteinlasskanal (12) mit einer Bluteinlassöffnung (13) ist, der nicht nur tangential, sondern auch schräg von oben nach unten in die Zentrifugierkammer (8) gerichtet ist, unter einem nach oben öffnenden Winkel (β) zur Rotationsachse (10) von weniger als 90°;
1.6 dass die Zentrifugierkammer (8) eine von der Bluteinlaßöffnung (13) am oberen Kammerende bis zur Blutauslassöffnung (15) am unteren Kammerende trichterartig enger werdende Form zur Reduzierung des Rotations-Energieverlustes des Saug-Blutstromes aufweist;
1.7 dass der Gasauslaß (23) mit einem Saugkanal (22) versehen ist zum Absaugen von Gas von der Oberfläche des in der Zentrifugiertcammer (8) rotierenden Blutstromes durch eine an den Saugkanal (22) anschließbare Saugvorrichtung (28);
1.8 dass ein Gasraum (18) vorgesehen ist, der sich von der Zentrifugierkammer (8) oberhalb ihrer Bluteinlaßöffnung (13) mindestens bis zu einer Gasauslaßöffnung (23) des Gasauslasses nach oben erstreckt und einen Querschnitt quer zur Rotationsachse (10) hat, der mindestens zweimal so groß ist wie der Querschnitt der Gasauslaßöffnung (23), und in welchem Gasblasen und Blut-Gas-Schaum Zeit und Raum haben, um in Blutteile und Gasteile zu zerfallen, bevor die Gasteile durch den Gasauslaß (23) von der daran angeschlossenen Saugquelle (28) abgesaugt werden.

2. Vorrichtung nach Anspruch 1, **dadurch gekennzeichnet, dass** an den Bluteinlaßkanal (12) eine Blutabsaugkanüle (36; 136; 236) angeschlossen ist oder der Bluteinlaßkanal (12) durch eine solche Blutabsaugkanüle gebildet ist.

3. Vorrichtung nach Anspruch 1 oder 2, **dadurch gekennzeichnet, dass** sie als ein mit einer Hand tragbares Gerät ausgebildet und mit einem Handgriffteil (4; 140; 240) versehen ist.

4. Vorrichtung nach Anspruch 3, **dadurch gekennzeichnet, dass** die Zentrifugierkammer (8) am vorderen oder oberen Ende des Handgriffteils (4) angeordnet ist, welches entgegengesetzt zur Blutströmungsrichtung gerichtet ist.

5. Vorrichtung nach Anspruch 3, **dadurch gekennzeichnet, dass** die Zentrifugierkammer (8) am hinteren oder unteren Ende, bezüglich der Blutstromrichtung stromabwärts des Handgriffteils (140) angeordnet ist.

6. Vorrichtung nach Anspruch 3, **dadurch gekennzeichnet, dass** die Zentrifugierkammer (8) in den Handgriffteil (240) integriert ist.

7. Vorrichtung nach einem der Ansprüche 3 bis 6, **dadurch gekennzeichnet, dass** der Handgriffteit (140) an der Blutabsaugkanüle (136) vorgesehen ist.

8. Vorrichtung nach einem der vorhergehenden Ansprüche, **dadurch gekennzeichnet, dass** die Blutauslaßöffnung (15) eine andere Querschnittsgröße hat als der Gasauslaß (23).

## Claims

1. Device for separating gas from blood, containing
1.1 a non-rotating centrifuging chamber (8) for producing a blood stream rotating about an upright, vertical or almost vertical axis (10) of rotation defined by the centrifuging chamber (8) like a cyclone with constant direction of rotation;
1.2 a blood inlet (12, 13) in the upper region and a blood outlet opening (15) in the lower region of the centrifuging chamber (8), in order to draw a blood stream by suction or the negative pressure of a suction device (28), which can be connected to the blood outlet opening (15), from a blood source via the blood inlet (12, 13) through the centrifuging chamber (8), in which it rotates about the axis of rotation, without reversing the blood stream direction of rotation through the blood outlet opening (15);
1.3 wherein the flow direction of the blood inlet (12, 13) is directed into the centrifuging chamber essentially tangentially to the axis (10) of rotation of the blood stream;
1.4 a gas outlet (23) arranged at a vertical interval higher than the blood inlet (12, 13);
**characterised in that**
1.5 the blood inlet is a blood inlet channel (12) having a blood inlet opening (13) which is directed into the centrifuging chamber (8) not only tangentially, but also at an angle from top to bottom, at an angle (β), opening at the top, to the axis (10) of rotation of less than 90°;
1.6 **in that** the centrifuging chamber (8) has a shape becoming narrower like a funnel from the blood inlet opening (13) on the upper chamber end to the blood outlet opening (15) on the lower chamber end, to reduce the rotational energy loss of the suction blood stream;
1.7 **in that** the gas outlet (23) is provided with a suction channel (22) for withdrawing gas from the surface of the blood stream rotating in the centrifuging chamber (8) through a suction device (28) which can be connected to the suction channel (22);
1.8 **in that** a gas chamber (18) is provided which extends upwards from the centrifuging chamber (8) above its blood inlet opening (13) at least as far as a gas outlet opening (23) of the gas outlet and has a cross-section transverse to the axis (10) of rotation, which is at least twice as large as the cross-section of the gas outlet opening (23), and in which gas bubbles and blood-gas foam have time and space in order to disintegrate into blood parts and gas parts, before the gas parts are withdrawn through the gas outlet (23) by the suction source (28) connected thereto.

2. Device according to claim 1, **characterised in that** a blood withdrawing cannula (36; 136; 236) is connected to the blood inlet channel (12) or the blood inlet channel (12) is formed by such a blood withdrawing cannula.

3. Device according to claim 1 or 2, **characterised in that** it is designed as an apparatus which can be supported by a hand and is provided with a handle part (4; 140; 240).

4. Device according to claim 3, **characterised in that** the centrifuging chamber (8) is arranged on the front or upper end of the handle part (4), which is directed opposite to the blood flow direction.

5. Device according to claim 3, **characterised in that** the centrifuging chamber (8) is arranged on the rear or lower end, with respect to the blood flow direction downstream of the handle part (140).

6. Device according to claim 3, **characterised in that** the centrifuging chamber (8) is integrated into the handle part (240).

7. Device according to one of claims 3 to 6, **characterised in that** the handle part (140) is provided on the blood withdrawing cannula (136).

8. Device according to one of the preceding claims, **characterised in that** the blood outlet opening (15) has a different cross-sectional size than the gas outlet (23).

## Revendications

1. Dispositif pour dissocier gaz et sang, comprenant :
1.1 une chambre de centrifugation non rotative (8) pour produire un flux de sang tournant à la manière d'un cyclone, dans un sens de rotation toujours constant, autour d'un axe de rotation (10), vertical ou presque vertical défini par la chambre de centrifugation (8) ;
1.2 une entrée de sang (12, 13) dans la partie supérieure et une ouverture de sortie du sang (15) dans la partie inférieure de la chambre de centrifugation (8), afin d'aspirer à travers l'ouverture de sortie du sang (15) un flux de sang, qui provient d'une source de sang et pénètre dans la chambre de centrifugation (8) par l'intermédiaire de l'entrée de sang (12, 13), par l'aspiration ou la dépression créée par un dispositif d'aspiration (28) pouvant être raccordé à l'ouverture de sortie du sang (15), sachant que, dans la chambre de centrifugation, le flux de sang tourne autour de l'axe de rotation sans inverser son sens de rotation ;
1.3 sachant que l'entrée de sang (12, 13) est orientée essentiellement tangentiellement à l'axe de rotation (10) du flux de sang dans la chambre de centrifugation ;
1.4 une sortie de gaz (23) située plus haut que l'entrée de sang (12, 13);
**caractérisé en ce que**
1.5 l'entrée de sang est un canal d'entrée du sang (12) comportant une ouverture d'entrée du sang (13), qui se dirige non seulement tangentiellement, mais également en biais de haut en bas dans la chambre de centrifugation (8) en formant avec l'axe de rotation (10) un angle (β) s'ouvrant vers le haut inférieur à 90° ;
1.6 la chambre de centrifugation (8) présente une forme allant en se rétrécissant à la manière d'un entonnoir depuis l'ouverture d'entrée du sang (13) au niveau de l'extrémité supérieure de la chambre jusqu'à l'ouverture de sortie du sang (15) au niveau de l'extrémité inférieure de la chambre, afin de réduire la perte d'énergie rotatoire du flux de sang aspiré,
1.7 **en ce que** la sortie de gaz (23) est dotée d'un canal d'aspiration (22) pour aspirer du gaz à la surface du flux de sang tournant dans la chambre de centrifugation (8) à l'aide d'un dispositif d'aspiration (28) pouvant être raccordé au canal d'aspiration (22),
1.8 **en ce qu'**est prévue une chambre à gaz (18) qui débute au-delà de l'ouverture d'entrée du sang (13) de la chambre de centrifugation (8) et s'étend vers le haut au moins jusqu'à une ouverture de sortie du gaz (23) de la sortie de gaz et présente une section transversale orientée transversalement par rapport à l'axe de rotation (10), qui représente au moins le double de la section transversale de l'ouverture de sortie du gaz (23), chambre dans laquelle des bulles de gaz et de la mousse de sang et de gaz ont la place et le temps pour se dissocier en particules de sang et en particules de gaz, avant que les particules de gaz ne soient aspirées à travers la sortie de gaz (23) à l'aide de la source d'aspiration (28) qui y est raccordée.

2. Dispositif selon la revendication 1, **caractérisé en ce qu'**une canule d'aspiration du sang (36 ; 136 ; 236) est raccordée au canal d'entrée du sang (12) ou **en ce que** le canal d'entrée du sang (12) est constitué par une telle canule d'aspiration du sang.

3. Dispositif selon la revendication 1 ou 2, **caractérisé en ce qu'**il est réalisé en tant qu'appareil pouvant être porté d'une seule main et est muni d'une poignée (4 ; 140 ; 240).

4. Dispositif selon la revendication 3, **caractérisé en ce que** la chambre de centrifugation (8) est située à l'extrémité avant ou supérieure de la poignée (4) qui est orientée en sens inverse par rapport au sens de circulation du sang.

5. Dispositif selon la revendication 3, **caractérisé en ce que** la chambre de centrifugation (8) est située à l'extrémité arrière ou inférieure, c'est-à-dire en aval de la poignée (140) par rapport au sens de circulation du sang.

6. Dispositif selon la revendication 3, **caractérisé en ce que** la chambre de centrifugation (8) est intégrée dans la poignée (240).

7. Dispositif selon l'une des revendications 3 à 6, **caractérisé en ce que** la poignée (140) est prévue sur la canule d'aspiration du sang (136).

8. Dispositif selon l'une des revendications précédentes, **caractérisé en ce que** la taille de la section transversale de l'ouverture de sortie du sang (15) est différente de celle de la sortie de gaz (23).
